# EUROPÄISCHE PATENTSCHRIFT

(11) **EP 1 329 434 B1**
(45) Veröffentlichungstag und Bekanntmachung des Hinweises auf die Patenterteilung: **31.08.2005**
(21) Anmeldenummer: 02090412.4
(22) Anmeldetag: 26.04.1999
(51) Int. Cl.: C04B 20/00, C04B 28/18

(54) **Verwendung von mikronisierten Materialien als Ziegelzuschlagstoff**
Use of micronized materials as an additive to bricks
Utilisation des matériaux micronisés comme agrégat pour briques

(43) Veröffentlichungstag der Anmeldung: 23.07.2003
(62) Teilanmeldung aus: 99913546.0
(73) Patentinhaber: Lelas, Tihomir, 10040 Zagreb (HR)
(72) Erfinder: Lelas, Tihomir, 10040 Zagreb (HR)
(74) Vertreter: Omsels, Hermann-Josef

(56) Entgegenhaltungen:
- EP-A- 0 112 999
- EP-A- 0 686 611
- WO-A-00/50362
- FR-A- 1 068 256
- "CHEMICAL ABSTRACTS + INDEXES, AMERICAN CHEMICAL SOCIETY. COLUMBUS, US" CHEMICAL ABSTRACTS + INDEXES, AMERICAN CHEMICAL SOCIETY. COLUMBUS, US, XP000154171 ISSN: 0009-2258

## Beschreibung

Die Erfindung betrifft ein Verfahren zur Herstellung von Ziegeln mit hoher Festigkeit.

Aus der EP 0 686 611 A1 ist ein formbares feuerfestes Material bekannt, welches aus einem feinen, Wasser gegenüber inaktiven, feuerfesten Puder, einem Aluminium enthaltenden Zement sowie feinem Silica-Puder besteht. Diese Material ist besonders als Beschichtung für Formen in einer Stahlgießerei geeignet, da es eine hohe mechanische Resistenz gegenüber heißen Flüssigkeiten aufweist. Daher werden die Oberflächen von beispielsweise Formen einer Gießerei nicht so leicht beschädigt und können weiter verwendet werden.

Die WO 00/50362 beschreibt eine Methode, um eine aushärtende zementhaltige Mischung herzustellen. Diese Mischung enthält neben de Zementpartikeln noch eine wässrige Lösung, welche einen Weichmacher und Puzzolane enthält. Bei Puzzolanen handelt es sich um anorganische Betonzusatzstoffe, welche kieselsäurehaltige oder kieselsäure- und tonerdehaltige natürliche oder künstliche Stoffe ohne selbstständiges Bindevermögen sind, die zusammen mit Wasser und Kalk wasserunlösliche Verbindungen mit zementartigen Eigenschaften bilden. Aufgrund ihres kleineren Durchmesser als die Zementpartikel bringen die Partikel des Puzzolans die Mischung zum Aushärten. Eine solche Mischung ist durchaus auch zur Klinkerherstellung geeignet. Eine Puzzolan-haltige Mischung für das Bauwesen wird ebenfalls in der FR 1 068 256 beschrieben.

Die EP 0 112 999 A1 beschreibt einen Schaumbetonstein und eine Verfahren zu seiner Herstellung. Der mineralische Zuschlagstoff eines solchen Schaumbetonsteins ist erfindungsgemäß kohlenstoffarm oder thermisch vom Kohlenstoff befreit. So wird es ermöglicht staubartige Sekundärstoffe bzw. Abgang des Steinkohlebergbaus für die Ziegelherstellung zu verwenden.

Eine Vorrichtung, die geeignet ist Mineralien derart zu zerkleinern, dass deren chemisches Potential vergrößert wird, ist aus der DE 197 55 921.2 bekannt. Die dortige Erfindung beschreibt ein Verfahren zur Verbesserung der Wirksamkeit von Wirkstoffen, die mindestens aus Mineralstoffen bestehen, indem diese Wirkstoffe einer tribomechanischen Aktivierung unterzogen werden, bei der die Oberfläche der behandelten Wirkstoffe vergrößert und deren Struktur zur Vergrößerung des chemischen Potentials destabilisiert wird. Die dazu beschriebene Vorrichtung weist mindestens drei Kranzreihen auf, die einander gegenläufig angetrieben werden, wobei auf jeder Kranzreihe schaufelartige Vorsprünge (Ventilatorschaufeln) befestigt sind. Insbesondere besteht die Vorrichtung zum Feinmahlen und Mikronisieren von Materialien aus einem Gehäuse mit zwei Rotoren, die jeweils mehrere, ineinandergreifende, einander gegenläufig mit gleicher Winkelgeschwindigkeit angetriebene Kränze aufweisen, die getrennt angesteuert werden, und wobei die Kränze hohl sind und in ihrem Innern eine Vielzahl von beidseitig an den Kranzwänden befestigten Ventilatorschaufeln tragen, die als Kollisionshindernis für die feinzumahlenden und mikronisierenden Materialien dienen, und wobei die Materialien aufgrund der im Inneren des Gehäuses herrschenden Zentrifugalkräfte von einem inneren Kranz in einen äußeren Kranz transportiert werden. Die Aktivierung der Mineralstoffe geschieht dadurch, dass in die Integrität der Kristallgitter eingegriffen wird, wodurch sich eine Art Beschädigung ergibt, die sich wiederum in Form einer Aktivierung, beispielsweise auch elektrischer Art, bemerkbar macht. Die DE 197 55 921.2 sieht dabei als vorteilhaft die Behandlung von Zeolithen an, die dort zum heilsamen Verzehr für Menschen beschrieben werden; auch Calcite für den Agrarbereich werden erwähnt. Die durch die bekannte Vorrichtung zu erzielende Mikronisierung liegt bei 20 µm pro Teilchen, wobei nur ca. 78 % aller Teilchen diese Größenordnung erreichten. Nachteile der DE 197 55 921.2 sehen wie folgt aus:
■ die dortige Rotoren bestehen aus Kränzen, in welche die Ventilatorschaufeln beidseitig eingebaut sind, daß heißt sich wie die Rippen in einem Skelett von einem zentralen Mittelpunkt aus verzweigen;
■ die dortigen Ventilatorschaufeln sind aus mehreren Bestandteilen zusammengeschweißt;
■ die dortigen Ventilatorschaufeln werden auf ihrer Oberkante sehr schnell abgenutzt - bedingt durch den Abreibprozeß. Daraus resultierte zwangsläufig eine Reduzierung der Rotorengeschwindigkeit; denn je schneller sich die Rotoren drehen, desto größer ist die Abreibung. Auch bedingt die Form der Ventilatorschaufeln eine erhöhte Anlagerung des Ausgangsmaterials, was wiederum zu einer Gewichtszunahme der Schaufeln führte und letztlich zu einem erhöhten Energieverbrauch.
■ die durch die bekannte Vorrichtung zu erzielende Mikronisierung liegt bei 20 µm pro Teilchen, wobei nur ca. 78 % aller Teilchen diese Größenordnung erreichten;

Ausgehend von diesem Stand der Technik ist es daher Aufgabe der vorliegenden Erfindung, ein Verfahren zur Herstellung von zementfreien Ziegeln mit hoher Festigkeit durch ein verkürztes thermisches Verfahren zur Verfügung zu stellen.

Gelöst wird diese Aufgabe durch die im Patentanspruch 1 aufgeführten Merkmale.

Erfindungsgemäß ist demnach ein Verfahren zur Herstellung von Ziegeln mit hoher Festigkeit vorgesehen, welches durch die folgenden Verfahrensschritte gekennzeichnet ist,
a) mikronisierter Wüstensand, welcher jeweils durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert wurde, wobei bis zu 28,36 % der Körner einen Durchmesser von weniger oder gleich 0,5 µm aufweisen, wird mit Kalk in einem Verhältnis von 0,906 - 0,954 : 0,046 - 0,094 (mikronisiertes Mineral:Kalk) zu einem Rohgemisch vermischt, wobei
b) anschließend dem Rohgemisch Wasser in einem Verhältnis 1 : 0,08 - 0,12 (Rohgemisch:Wasser) zugegeben wird, und
c) durch einen Druck zwischen 40 - 90 MPa und einer Temperatur zwischen 90 - 170 °C zwischen 4,5 und 7 Stunden ein Ziegel ausgehärtet wird.

Die erfindungsgemäß verwendete Vorrichtung zum Mikronisieren der Rohstoffkomponente weist einen höheren Wirkungsgrad bei der Mikronisierung auf. Dies wird dadurch erreicht, daß die Rotoren aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern. Durch diese Maßnahmen wird eine wesentlich effizientere Mikronisierung erreicht unter gleichzeitiger Schonung der Vorrichtung selbst. Daß heißt, die üblicherweise mit der Feinmahlung einhergehende starke Abnutzung der Ventilatorschaufeln und damit der gesamten Kranzreihen, was zu erhöhtem Wartungsbedarf führt und sehr kostenintensiv ist, wird vermieden. Erreicht wird dies dadurch, daß durch die Kanäle die Abnutzung der Ventilatorschaufeln selbst minimiert wird, indem bewußt ein Verbleiben von mikronisiertem Material innerhalb der Kanäle in Kauf genommen wird, was wiederum zu einem erhöhten Widerstand an den Ventilatorschaufeln selbst führt und damit letztlich zu einem erhöhten Grad der Mikronisierung.
■ Die Vorteile der erfindungsgemäß verwendeten Vorrichtung lassen sich wie folgt darstellen:Diese Feinmahlung wird durch kontrollierte Luftströmungen erzielt, die durch die Anwendung von neuartig konstruierten Ventilatorschaufeln hervorgerufen werden.
■ Durch die Auswahl der Einstellung und der Neigung der Ventilatorschaufeln wird eine turbulente Luftströmung erzeugt, welche die Effizienz des Mikronisierungsprozesses steigert. Die neu konstruierte Form dieser Ventilatorschaufeln verlängert deren Lebensdauer im Verhältnis zu den bekannten Ventilatorschaufeln bis zu 30 mal.
■ Durch den einfachen Austausch von abgenutzten Werkzeugen (Ventilatorschaufeln und Stiften) ist das Handling und die Instandhaltung vereinfacht. Bei herkömmlichen Einrichtungen war dies bislang nicht möglich. Es mußten immer ganze Kränze ausgetauscht werden.
■ Die Kanäle, in welchen die Ventilatorschaufeln durchgreifen, bilden ein geschlossenes Labyrinthsystem für die Materialverarbeitung, welches die Bewegung des Materials, das der Verarbeitung unterzogen ist, so kontrolliert, daß die Körnchen nicht ohne Schlag- und Reibungswirkung neben den Ventilatorschaufeln unverarbeitet vorbeifliegen, womit die Effizienz der Verarbeitung optimiert wird.
■ Die zu erzielende Mikronisierung liegt bei 98,72 % aller Teilchen unter 4,3 µm. Ein Anteil von 28,36 % aller Teilchen weist sogar einen Durchmesser von unter 0,5 µm auf. Mit keinem herkömmlichen Verfahren oder einer bekannten Vorrichtung konnten derartige Ergebnisse erzielt werden.

Die erfindungsgemäß verwendetet Vorrichtung zur Zerkleinerung ruft bei mineralischen Rohstoffkomponenten diverse chemische und chemischphysikalische Veränderungen hervor. Die Effekte, welche durch dynamische Reibungsprozesse entstehen, verleihen diesen Mineralen neue Eigenschaften, die sich bei der Herstellung diverser Produkte technologisch und kommerziell nutzen lassen.

Die verwendete Vorrichtung zur Zerkleinerung weist Vorteile bei der Behandlung von Rohstoffkomponenten aller Art, insbesondere zur Verwendung in der Baubranche auf, nämlich bei
■ Sand aller Art, Wüstensand eingeschlossen
■ Ton aller Art
■ Kalksteine
■ Tuff und Tuffelsen
■ Asche und Lavaschlacke
■ Kies, usw.

Ferner sind mineralische Rohstoffkomponente sekundärer Herkunft ebenfalls bevorzugt:
■ Industrieschlacke und -asche
■ Asche und Pulver elektronischer Filter
■ Baumüll, -schutt
■ ausgesonderte Ziegel
■ Tonabbrand
■ Magnesitabraum, usw.
Im Vergleich mit nichtaktiviertem Material, zeigt fein gemahlenes und mikronisiertes Material mit der verwendeten Vorrichtung bearbeitet, eine erhöhte freie Energie und eine erhöhte Reaktionsfähigkeit. Bevorzugt ist eine Korngröße des unbearbeiteten Materials zwischen 0 - 600 µm.

Für die Verbesserung der Bindemittel wird dem bearbeiteten Material eine geringe Menge hydratisierten oder ungelöschten Kalks beigegeben.

Durch Homogenisierung fein gemahlener und mikronisierter Rohstoffkomponenten (für den beschriebenen Versuch wurde Wüstensand aus Dubai ausgewählt), sowie hydratisierten Kalk und Wasser ist eine Mischung erhältlich, die durch Pressen oder Vibrieren in Schablonen geformt werden kann, die für diese Zwecke ausgesucht sind und dann durch das Verfahren der hydrothermalen Bearbeitung unter folgenden Bedingungen trocknet werden:
■ im Wasserdampf bei einer Temperatur bis 90° C
■ unter Bedingungen erhöhten Drucks mit Wasserdampf bei einer Temperatur bis 170° C

Dieses Verfahren der Herstellung von Baumaterial basiert auf der beschleunigten Reaktion zwischen SiO₂ und Al₂O₃ aus der Zusammensetzung des Baumaterials auf der einen Seite und Ca(OH)₂ aus Kalk auf der anderen.

Die Anteile der Zusammensetzung technologischer Parameter und die Eigenarten der fertigen Produkte, können mit folgenden Angaben beschrieben werden: Der Anteil an feingemahlenem und mikronisiertem Wüstensand beträgt 90,6 - 95,4 %; der Kalkanteil im Gemisch 4,6 - 9,4 %. Das Gemisch wird homogenisiert und Wasser wird im Verhältnis 1,0:0,08-0,12 (Gemisch:Wasser) dazugegeben. Das so vorbereitete Rohgemisch wird durch Pressen geformt; anschließend härtet es unter folgenden Bedingungen hydrothermal aus: Druck des Pressens: 40 - 90 MPa; Temperatur der Aushärtung: 90 und 170°C; Dauer der Aushärtung: 4,5 - 7,0 h.

### Ausführungsbeispiel

### a) Herstellung eines Ziegels bei einer Temperatur von 90°C:

Ein Ziegel der Größe 25,0 x 12,5 x 6,5 cm wird aus 3,779 kg erfindungsgemäß fein gemahlenen und mikronisierten Wüstensand, 0,200 kg hydratisierten Kalk und 0,400 kg Wasser hergestellt. Die Eigenschaften des späteren Ziegels resultieren aus dem Druck des Pressens, aus der Temperatur und der Dauer der Aushärtung:
■ Druck des Pressens 40 MPa
■ Temperatur der Aushärtung 90°C
■ Dauer der Aushärtung 4,5 h

Der Ziegel, der auf diese Weise hergestellt wurde, hat folgende Kennzeichen:
■ Festigkeit auf Druck
■ nach einem Tag 20,6 MPa
■ nach 28 Tagen 28,6 MPa
■ Volumenmasse 2040 kg/m³
■ Wasseraufnahme 15,80 %

### b) Herstellung eines Ziegels bei einer Temperatur von 170°C (Aushärtung im Autoklav):

Ein Ziegel der Größe 25,0 x 12,5 x 6,5 cm wird aus 3,779 kg erfindungsgemäß fein gemahlenen und mikronisierten Wüstensand, 0,200 kg hydratisierten Kalk und 0,400 kg Wasser hergestellt. Die Eigenschaften des späteren Ziegels resultieren aus dem Druck des Pressens, aus der Temperatur und der Dauer der Aushärtung:
■ Druck des Pressens 90 MPa
■ Temperatur der Aushärtung 170°C
■ Dauer der Aushärtung 7,0 h

Der Ziegel, der auf diese Weise hergestellt wurde, hat folgende Kennzeichen:
■ Festigkeit auf Druck
■ nach einem Tag 36,2 Mpa
■ nach 28 Tagen 38,4 Mpa
■ Volumenmasse 2040 kg/m³
■ Wasseraufnahme 14,50 %

Die gewünschten physikalisch-mechanischen Eigenschaften des Materials hängen unmittelbar vom beschriebenen Bearbeitungsverfahren, sowie auch von den ausgewählten technologischen Parametern, die beim Herstellungsverfahren angewendet werden, ab. Vergleichbar mit diesen Ergebnissen sind allenfalls Betonziegel, welche durch Zugabe von Zement (15-25 Gew.-%) hergestellt werden. Die erfindungsgemäße hergestellten Ziegel weisen keinerlei bauphysikalische Nachteile auf.

Ziegel, Dachziegel, Balken, Blöcke, Töpfe und andere keramische Produkte werden herkömmlicherweise aus solchen Tonarten hergestellt, in denen sich nicht verschiedene Bestandteile befinden, die dem Backverfahren der geformten Produkte schaden. So ruft zum Beispiel der erhöhte Anteil an Kalziumcarbonaten (CaCO₃) in der Beschaffenheit der Rohstoffkomponente Ton ein sogenanntes Aufblühen auf dem fertigen Produkt hervor, da sich die Backtemperatur im Temperaturbereich des Sinterprozesses erhöht (in einem Bereich von 960 - 1200°C).

Überraschenderweise wurde gefunden, daß es bei der Behandlung von Ton in der erfindungsgemäßen Vorrichtung zu einem gravierenden "Schaden" innerhalb des Kristallgitters kommt, was zu einer mechanisch-bedingten Aktivierung der Oberfläche und der Struktur und zum Übergang eines Teiles der Kristallphase in eine amorphe Form und überhaupt zur Änderung des energetischen Zustandes des Rohstoffes führt. Es konnte nachgewiesen werden, daß dadurch Ton seine physikalischen und chemisch-physikalischen Eigenheiten ändert, was sich wiederum in einer Änderung des Temperaturbereichs des Backens ändert; auch verringert sich der Punkt des Sinterprozesses deutlich und das gesamte Verfahren der thermischen Bearbeitung wird verkürzt. Zum Nachweis hierfür wurde Ton mit einem erhöhten Anteil an CaCO₃ (3,8 %) eingesetzt, welcher normalerweise nicht für die Herstellung von Backsteinen benutzt wird. Der Rohstoff wurde in der erfindungsgemäßen Vorrichtung mikronisiert und feingemahlen. Das so vorbereitete Material wurde mit Wasser gemischt, bis es die optimale Feuchtigkeit erreicht hat und wurde anschließend durch das Pressverfahren geformt. Es wurde ein Backstein normaler Größe 6,5 x 12,5 x 25,0 cm angefertigt. Der Backstein wurde nach der Formung durch das Pressverfahren bei einer Temperatur bis 200°C 24 Stunden getrocknet und anschließend bei einer Temperatur von 560°C gebacken. Bei dieser Temperatur ist es gelungen, den Sinterprozeß durchzuführen. Diese Temperatur wurde 60 Minuten gehalten, nachdem der Abkühlungsprozeß Schrittweise begonnen hatte. Der gesamte Vorgang der Erhöhung der Temperatur, des Sinterprozesses, sowie der Abkühlung, dauerte 12 Stunden.

Als Kontrolle wurde ein Backstein normaler Größe 6,5 x 12,5 x 25,0 cm benutzt; als Rohstoffbasis wurde die gleiche Rohstoffkomponente Ton verwendet wie im zuvorigen Experiment, mit dem Unterschied, daß der Ton nicht mikronisiert wurde. Der Backstein mußte nach der Formung durch das Pressverfahren bei einer Temperatur von 240°C 48 Stunden getrocknet werden. Danach wurde er bei einer Temperatur bis 980° C gebacken, bei welcher der Temperaturbereich des Sinterprozesses erreicht wurde. Diese Temperatur mußte 120 Minuten gehalten werden, bis es möglich war, den Abkühlungsprozeß einzuleiten. Der ganze Vorgang der Erhöhung der Temperatur, des Sinterprozesses und der Abkühlung, dauerte 22 Stunden. Der Vorgang wurde 25 mal wiederholt, ohne daß es zu erheblichen Änderungen des Vorganges kam. Hinsichtlich der Qualität der hergestellten Muster wurden folgende Unterschiede festgestellt:

**Tabelle 9**

| Eigenschaft | Ziegel mit erfindungsgemäß bearbeitetem Ton | Ziegel mit normalem, unbearbeitetem Ton |
|---|---|---|
| Druckfestigkeit | 159 Kp/cm² | 83 Kp/cm² |
| Wasseraufnahme | 3,2 % | 5,4 % |
| Ausblühen | NEIN | JA |

Weitere vorteilhafte Maßnahmen sind in den übrigen Unteransprüchen enthalten. Die Vorrichtung zur Zerkleinerung der jeweiligen Rohstoffkomponente ist in den anliegenden Zeichnungen dargestellt und wird nachfolgend näher beschrieben. Es zeigt:
- **Figur 1**: eine schematische Ansicht der erfindungsgemäßen Vorrichtung;
- **Figur 2**: einen vertikalen Schnitt durch die Vorrichtung;
- **Figur 3**: eine Rotorenscheibe;

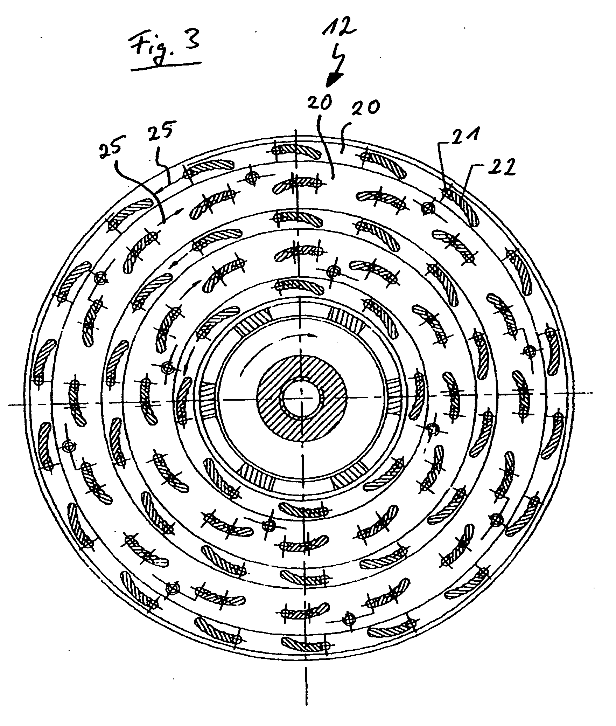

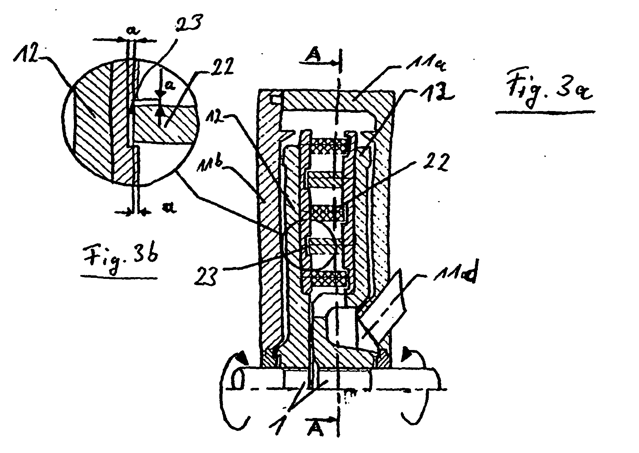

- **Figur 3 a**: einen vertikalen Schnitt durch einen Ausschnitt der zusammengesetzten Vorrichtung,
- **Figur 3b**: eine Ausschnittvergrößerung entlang eines Schnittes A-A gemäß der Fig. 3a;

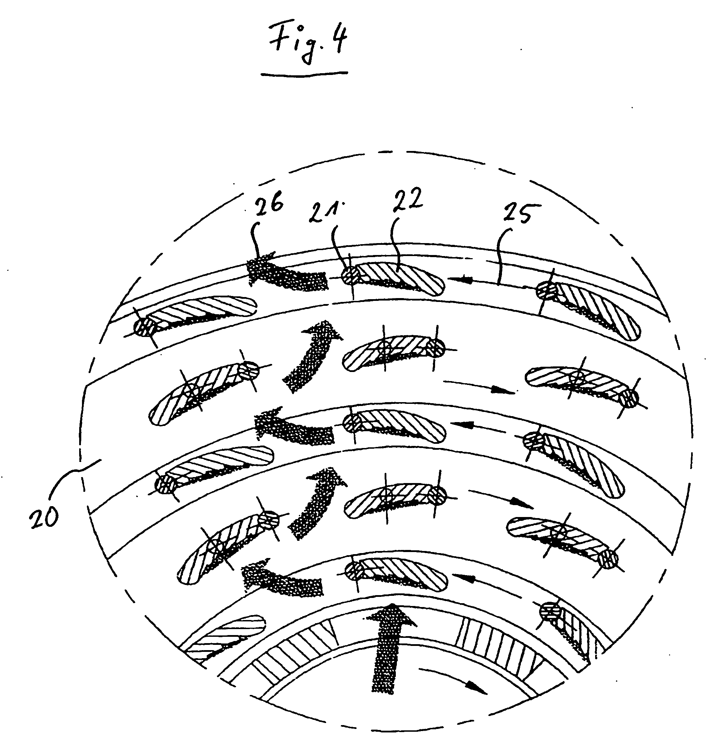

- **Figur 4**: die schematische Andeutung der Luftströme entlang der Ventilatorschaufeln;

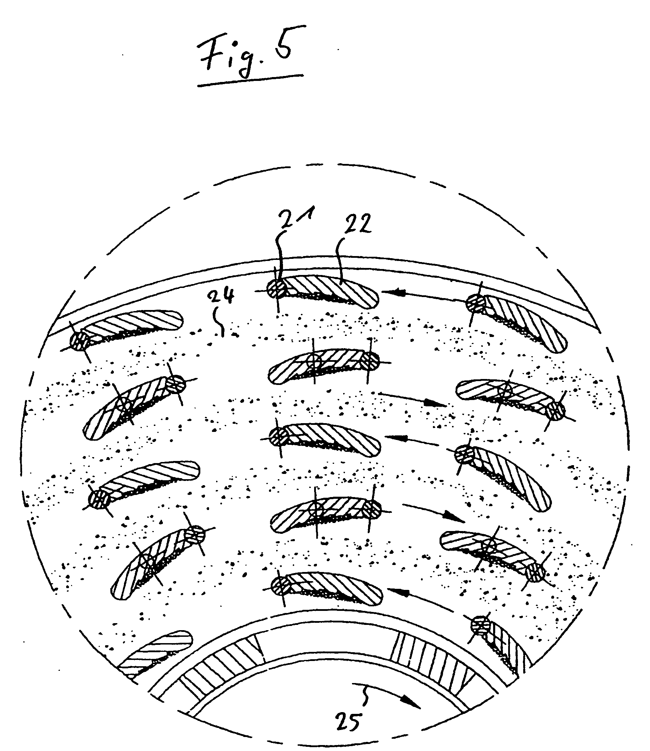

- **Figur 5**: die schematische Andeutung des zerkleinerten Materials entlang der Ventilatorschaufeln;

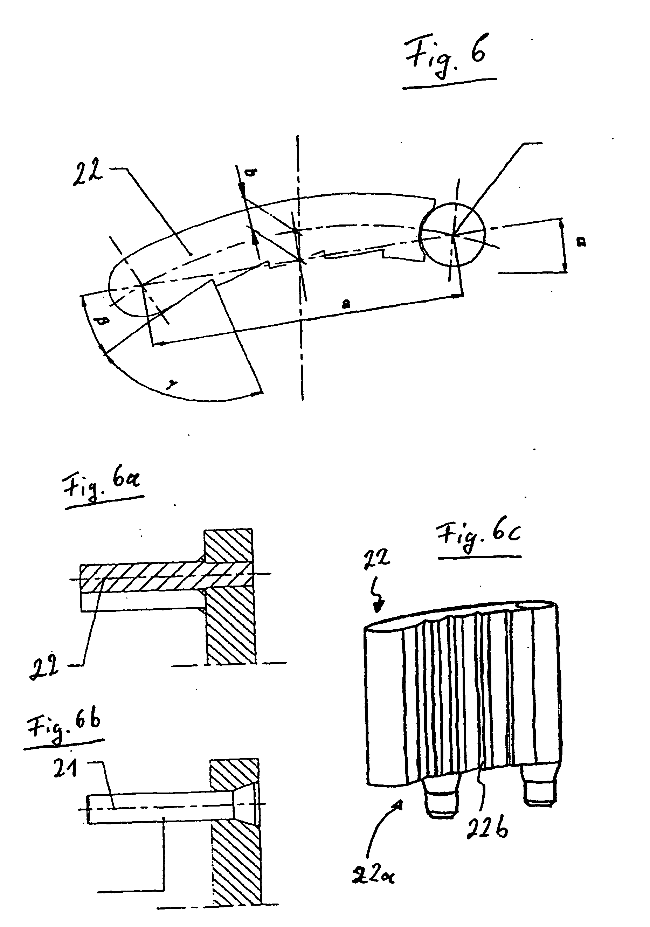

- **Figur 6**: eine Ventilatorschaufel mit Stift/Kanal im Detail und in mehreren Ansichten **(6a, 6b, 6c);**

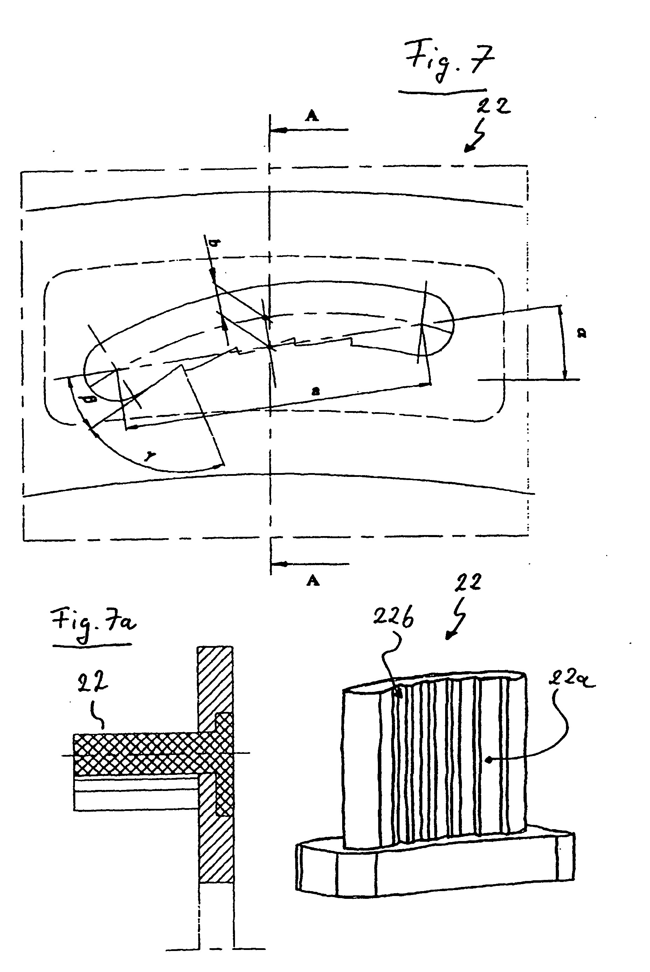

- **Figur 7**: schematische Darstellung einer Segmenten-Ventilatorschaufel;
- **Figur 7a**: Ausschnitt entlang eines Schnittes A-A gemäß Figur 7.

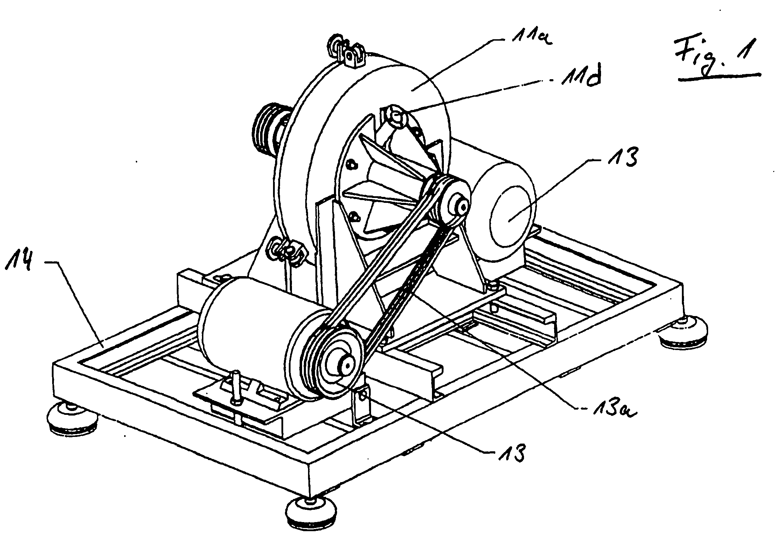

Die Figur 1 zeigt eine Vorrichtung 10 für die Feinmahlung und Mikronisierung, sowie das Homogenisieren von diversen festen und flüssigen Rohstoffkomponenten. Das Prinzip sieht derart aus, daß das Ausgangsmaterial durch die Mitte der Rotoren in den Verarbeitungsraum der Vorrichtung eingesaugt wird. Der Eintritt wird durch die Einwirkung von zentrifugalen Kräften in den Raum zwischen den Ventilatorschaufeln begünstigt und wird aufgrund der dort herrschenden Luftströme beschleunigt; so daß das Material mit dem bereits verarbeiteten Material kollidiert. Das Ausgangsmaterial wechselt die Bewegungsrichtung in sehr kurzen Intervallen; infolgedessen wird es zerkleinert und mikronisiert.

Die Vorrichtung 10 besteht aus einem aufklappbaren Gehäuse 11 mit einem Materialeinfuhrschacht 11d, in welchem sich zwei Rotorenscheiben 12 befinden, die gegeneinander gestellt sind und mittels entsprechender Motoren 13 über Riemen 13a gegenläufig betrieben werden, so daß sie sich mit gleicher Winkelgeschwindigkeit drehen. Das Gehäuse 11 und die Motoren 13 sind auf dem Fundament 14 befestigt und bilden eine unabhängige Einheit.

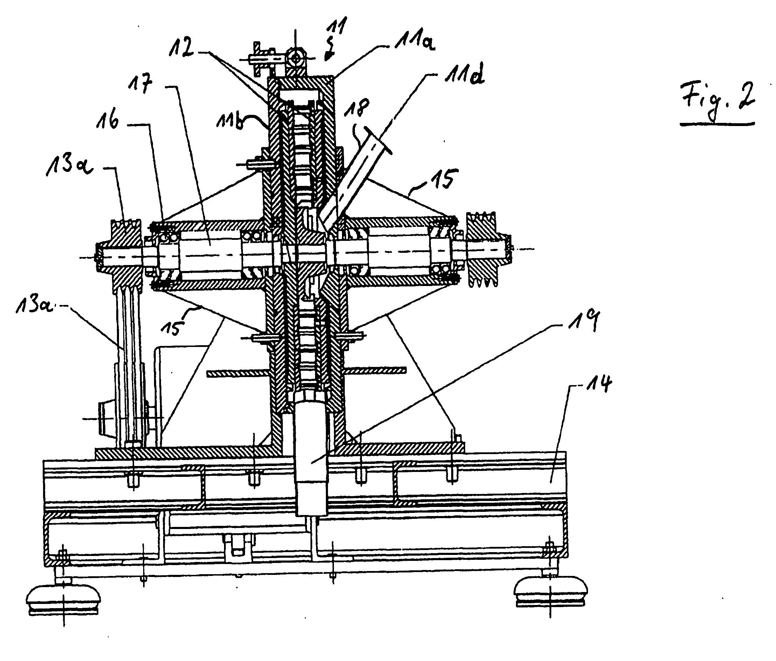

Figur 2 zeigt, daß das Gehäuse 11 aus zwei Teilen aufgebaut ist: eine Gehäuseseite 11a für die Materialeinfuhr und eine weitere Gehäuseseite 11b mit Einfuhrschacht 11c. Diese zwei Seiten 11a, 11b sind miteinander verschraubt. Auf beiden Seiten des Gehäuses 11 befinden sich die Träger 15, in welche die Lager 16 und Reckstangen 17 eingebaut sind. Auf der Seite 11a für die Materialeinfuhr befindet sich ein Rohr 18 für die geregelte Einfuhr des Materials; auf der unteren Seite befindet sich eine Öffnung 19 für den Ausstoß des fertigen Materials.

Figur 3, 4 und 5 zeigt, daß auf den Rotorenscheiben 12 mehrere konzentrische Kränze 20 mit den Stiften 21 und Ventilatorschaufeln 22 angeordnet sind, welche so konstruiert und ausgerichtet sind, daß sie berührungsfrei, während sie gegenläufig drehen, nebeneinander vorbeilaufen können - angedeutet durch die Drehrichtung 25. Minimal sind zwei Kränze erforderlich, die von den zwei Rotoren angetrieben werden. Die Aufgabe der Schlagstifte 21 und der Ventilatorschaufeln 22 ist die Erzeugung von turbulenten Luftströmungen für die Beschleunigung des verarbeiteten Materials, so daß Stöße und Reibung unter den Körnchen bei bestimmten Winkeln unter dynamischen Bedingungen hervorrufen werden. Die Kanäle 23 auf den Scheiben verhindern den Materialdurchgang unter den Ventilatorschaufeln 22, vgl. Figur 3 a und 3b.

Das Ausgangsgranulat (nicht dargestellt) wird, durch den Zentralteil 18 des Motorensystems durch Einsaugung eingebracht, durch die Luftströme 26 beschleunigt und so gesteuert, daß die Körnchen infolge mehrmaliger Bewegungsrichtung untereinander kollidieren und sich in sehr kurzen Zeitintervallen aneinander reiben. Dabei berühren sich die Arbeitswerkzeuge und andere Teile der Vorrichtung nicht oder nur geringfügig - aber es kommt auf keinen Fall zu Zerstörungen der Werkzeuge. Es entsteht eine Interaktion unter den Körnchen mit solchem Ausmaß, daß bei den Körnchen die innere Energie ausgetauscht wird, da die Zusammenstöße unplastisch sind (vgl. Fig. 4 und 5).

Die Effekte, die Folge der Zusammenstöße der Körnchen sowie Folge der relativen Bewegung der Oberfläche eines Körnchen über der Oberfläche von einem anderen Körnchen (mechanische Reibung) sind, werden verstärkt durch diejenigen Effekte, die infolge plötzlicher Richtungswechsel der Kömchenbewegung entstehen, so daß die Energie der Beschleunigung und relativer Bewegung der Körnchen in Energie der Deformierung, sowie in die Energie der molekularen Bewegung umgewandelt wird. Während der Zusammenstöße und der Reibung der Körnchen, welche dem Verkleinerungsprozeß in sehr kurzen Zeitintervallen (10⁻⁵ bis 10⁻⁶ s) unterzogen sind, entsteht eine bedeutende Veränderung ihrer Geometrie bzw. Form und Größe. Durch die relative Bewegung eines Körnchens über der Oberfläche eines anderen Körnchens, entstehen Schäden und Deformierungen der Kömchenoberfläche, sowie der Materialschichten, welche sich unmittelbar unter der Kornoberfläche befinden. Dadurch wird die Struktur des Kristallgitters auf der Oberfläche zerstört oder geschädigt, so daß teilweise die Kristallform in eine amorphe Phase umgewandelt wird, mit dem Ergebnis, daß die physikalischen und physikalisch-chemischen, sowie energetischen Eigenschaften des verarbeitenden Materials verändert werden. Bei solchen Verarbeitungen von Rohstoffkomponenten organischen Ursprungs zerreißen beispielsweise Zellulosefasern und große Molekülen werden mithin zu kleineren Molekülen, wobei diverse Veränderungen chemischer Zusammensetzung bei dem verarbeiteten Material hervorgerufen werden, sowie auch physikalische Veränderungen, die bedeutend für ihre weitere Materialverarbeitungen und/oder Aufbereitung, aber auch für die Wirksamkeit sind.

Neben der Veränderung der Eigenschaften des Materials, welches mit den beschriebenen Verfahren verarbeitet wird, wird dieses infolge von mechanischen Beanspruchungen fein gemahlen und mikronisiert und die Veränderung der granulometrischen Zusammensetzung des Materials ist von der Granulometrie der Ausgangskörner, sowie vom Niveau der Körnchenbeschleunigung, der geplanten Winkel der Zusammenstöße und gegenseitigen Reibung, sowie die geplanten Anzahl der Zusammenstöße abhängig. Folgende Parameter für die Feinmahlung und Mikronisierung mit der erfindungsgemäßen Vorrichtung stellen eine optimale Konfiguration dar, so daß diese Parameter bevorzugt sind:
■ Granulation des Ausgangskoms < 4,0 mm
■ Anzahl der Gesamtzahl der Kränze/Kranzreihen auf den Rotorenscheiben: 5
■ Durchmesser der Rotoren 500 mm
■ Drehzahl der Rotoren 3600 /Min.
■ Kapazität der Vorrichtung 300Kg/Std.

Verglichen mit den Materialien, welche auf herkömmliche technische Weise (vgl. DE 197 55 921.2) feingemahlen und mikronisiert werden, zeigt das Material, welches durch die erfindungsgemäße Vorrichtung feingemahlen und mikronisiert wurde, eine erhöhte freie Energie und Reaktionsfähigkeit.

Die Innovation liegt mithin in der Konstruktion (Form, Verzahnung, Neigung) und Austauschbarkeit von Stiften und Ventilatorschaufeln auf die konstruierten Kanäle, die sich auf der Rotorenscheibe befinden, sowie in der Auswahl des Materials für die Ausarbeitung von Ventilatorschaufeln. Die Rotationsscheiben der Vorrichtung rotieren mit der gleichen Winkelgeschwindigkeit, bewegen sich jedoch gegenläufig. Das Ausgangsmaterial wird durch das Einsaugrohr 18 in den Zentralteil der rotierenden Scheibe eingeführt, aufgrund von Zentrifugalkräften werden die Körnchen der Rohstoffkomponente in Richtung des äußeren Randes des Gehäuses beschleunigt. Die Körnchen schlagen auf die Kränze 20 der Ventilatorschaufeln 22, welche sich in Gegenrichtung drehen. Sie wechseln die Bewegungsrichtung aufgrund der Richtungswechsel der Ventilatorschaufeln. Ferner schlagen und reiben sich die Körnchen untereinander, gehen nachfolgend in einen weiteren Kranz 20 mit Ventilatorschaufeln 22 über, wechseln wieder die Richtung der Bewegung im Einklang mit dem Richtungswechsel der rotierenden Scheibe, solange bis sie das Schaufelsystem verlassen. Am Ende der Bearbeitung in der erfindungsgemäßen Vorrichtung schlagen die Körnchen auf der Wand des Gehäuses auf und werden durch die Ausgangsöffnung 19 abtransportiert.

Figur 3a und 3b zeigt, daß die Kanäle 23 auf den Scheiben 12, in welche die Ventilatorschaufeln 22 durchgreifen, den Materialdurchgang unter den Ventilatorschaufeln 22 verhindern. Deren Form wird gemäß den Eigenschaften der jeweils zu verarbeitenden Rohstoffkomponenten (Materials) - also Granulation des Ausgangsmaterials, seine Feuchtigkeit, Härte, Ursprung, chemische Zusammensetzung und ähnliches - definiert. Wenn z. B. die Eingangsgranulation des Materials < 1 mm beträgt, bedeutet dies, daß der minimale Abstand zwischen den Ventilatorschaufeln und den Kanälen auf den Scheiben größer als 1 mm sein soll, um überhaupt das Durchgehen des Materials zu ermöglichen. Bei der Ausarbeitung und der Montage der Scheiben soll zufriedenstellende Parallelität verwirklicht werden, um den Ventilatorschaufeln das Durchgreifen in die Kanäle zu ermöglichen, denn die Durchmesser der Scheiben sind relativ groß (500 mm). Vorteile gegenüber der in der DE 197 55 921.1 beschriebenen Vorrichtung liegen darin, daß die Ventilatorschaufeln aufgrund ihrer Form, Neigung und Verzahnung bei dem Prozeß der Feinmahlung und Mikronisierung mit einer feinen Schicht des verarbeiteten Materials belegt werden und auf diese Weise von der Schlag- und Reibungswirkung des Eingangsmaterials geschützt werden. Die Abnutzung der Oberfläche der Ventilatorschaufeln wird somit minimiert und die Lebensdauer bedeutend verlängert. Ferner wird die energetische Ladung des Materials, welches behandelt wird, angehoben, wenn die Körnchen untereinander kollidieren und nicht mit den Teilen der Ventilatorschaufeln. Die technologischen Parameter, wie die Anzahl der Ventilatorschaufeln, ihre Neigung, die Form der Schaufelverzahnung, Anzahl der Ventilatorkränze, Winkelgeschwindigkeit der Scheiben, definieren die späteren Eigenschaften des verarbeiteten Materials. Mit der Kombination der angeführten Parameter wird es möglich, die Ergebnisse und Effekte zu programmieren.

In der Figur 3b sind weiter Scheibenkanäle (23) näher dargestellt. In den Rotorenscheiben (12) befinden sich an denjenigen Orten, die mit der Kranzreihe (20) des gegenüberliegenden Rotors (13) korrespondieren, Ausnehmungen (20a; siehe Fig. 3a, 3b), innerhalb derer die Ventilatorschaufeln (22) des gegenüberliegenden Kranzes (20) laufen. Die Kanäle (23) decken sich gegenseitig mit der Länge a, welche 2 - 5 mm beträgt. Sie bilden quasi ein Labyrinth, welches den Durchgang des Materials unter den Ventilatorschaufeln verhindert. Durch die Entstehung des Labyrinthes verstärkt sich der Widerstand für die Strömung unter den Ventilatorschaufeln. Somit wird erreicht, daß sich die Körnchen des Ausgangsmaterials durch die Hauptströmung zwischen den Ventilatorschaufeln bewegen. Die Form der Kanäle wird gemäß technisch-technologischer Eigenschaften der Rohstoffkomponenten des verarbeiteten Materials definiert (Granulation des Ausgangsmaterials, seine Feuchtigkeit, Härte, Ursprung, chemische Zusammensetzung und ähnliches). Wenn es die Scheibenkanäle nicht geben würde, würde sich das Ausgangsmaterial aufgrund der Zentrifugalkräfte so bewegen, daß es vom Zentrum bis zu der Peripherie der Scheiben an den Ventilatorschaufeln und der Scheibe vorbeigeht. Bei der Ausgangsgranulation des Materials von 0 - 1 mm muß die Entfernung zwischen den Ventilatorschaufeln und den Kanälen größer als 1 mm sein, um den Durchgang des Materials überhaupt zu ermöglichen, vorteilhaft hat sich eine Entfernung von 2 mm genauso wie für die Verdeckung a erwiesen.

Die Figuren 6, 6a, 6b und 6c zeigen die Geometrie der Ventilatorschaufeln 22 und der Stifte 21. Der Kranz 20, die Ventilatorschaufeln 22 und die Stifte 21 sind aus Hartstahl gebaut. Eine Alternative ist, den Stift aus Porzellan und die Ventilatorschaufeln aus Stahl herzustellen. Die Neigung der Ventilatorschaufeln 22 beträgt im Verhältnis zur Horizontalen α = 4 - 15° mit der Optimierung auf 8 - 10°. Die Anordnung und die Größe von der Verzahnung ist von der Anzahl der Ventilatorschaufeln abhängig (β = 30 - 120° und γ = 60 - 120°). Der Biegungsgrad der Ventilatorschaufeln ist mit dem Verhältnis der Längen a und b definiert, wobei b 10 % der Sehnenlänge darstellt. Die Anordnung der Ventilatorschaufeln ist fixiert. Sie sind in die Kranzform in entsprechende Ausnehmungen eingepreßt Die Form der Ventilatorschaufeln, profilierte Kollisionsoberfläche 22a, 22b und die Neigung sichern die Abfüllung der Ventilatorschaufeln mit dem Ausgangsmaterial und werden damit von der Abnutzungswirkung des Ausgangsmaterials geschützt, was auf die Verlängerung der Lebensdauer der Ventilatorschaufeln wirkt. Die Zähne 22b auf den Ventilatorschaufeln halten die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Bevor die Ventilatorschaufeln 22 in die jeweilige Kranzreihe 20 der Rotorenscheibe 12 eingebracht wird, wird der Stift 21 in die Rotorenscheibe 12 eingepreßt. Die Symmetrieachse des Stiftes 21 befindet sich in der Symmetrieachse des Biegungsgrades der Ventilatorschaufeln 22, damit die Geometrie des Systems der Ventilatorschaufeln/Stifte optimal erreicht wird. Während der Materialverarbeitung schlagen die Materialkörnchen auf die Stirnseite der Ventilatorschaufeln und insbesondere auf und in die Stifte. Der Stift wird somit abgenutzt. Nach der Abnutzung wird der Stift durch Auspressen oder Bohren durch einen neuen ausgetauscht. Dies hat einen entscheidenden Vorteil: Bisherige Varianten der Ventilatorschaufeln ohne Stifte haben die Abnutzung der Ventilatorschaufeln selbst ergeben. Der Austausch der abgenutzten Ventilatorschaufeln ist im Vergleich mit dem Austausch der Stifte sehr kompliziert, aufwendig und teuer. Die Stifte können daher leicht ausgetauscht werden; die Ventilatorschaufeln bleiben unbeschädigt und müssen es nicht. De Austausch der Ventilatorschaufeln erfolgt erst bei allgemeiner Materialermüdung.

Das Problem der Vibrationen und der Festigkeit der Stifte ist dadurch gelöst, daß sich der Stift 21 mit der Fläche von 1/3 seines Stiftumfanges an eine korrespondierende Ausnehmung der Ventilatorschaufel 22 an diese Ventilatorschaufeln "anlehnt", und sich nicht geradflächig an die Ventilatorschaufeln anlehnt. Die Ventilatorschaufeln 22 werden durch Kaltpressen verformt oder auch durch Schmieden, wobei das Schmieden der besseren Aushärtung dient.

Eine andere Ausführungsform sieht erfindungsgemäß auch Segment-Ventilatorschaufeln (Figur 7, 7a) vor. Die Segment-Ventilatorschaufeln 22 werden aus Keramik oder Gußstahl hergestellt. Bei dieser Ausführungsform werden in die Rotorenscheiben 12 die Kanäle eingebaut, in welche die Segment-Ventilatorschaufeln mit unbestimmten Einlegungen gestellt werden. Die Genauigkeit der Einstellungen und die Festigkeit der Ventilatorschaufeln wird durch das Profil der Kanäle und durch die Reibung zwischen den Segment-Ventilatorschaufeln und die tragende Scheibe bestimmt. Die Neigung der Ventilatorschaufeln in Bezug auf die Horizontale beträgt α = 4 - 15° mit der Optimierung bei 8° - 10°. Die Einordnung der Zähne 22b und ihre Größe ist von der Länge der Ventilatorschaufeln 22 abhängig, Winkel β beträgt 30° - 120°, und Winkel γ beträgt 60° - 120°. Der Biegungsgrad der Ventilatorschaufeln ist mit dem Verhältnis der Längen a und b definiert, wobei b 10 % der Sehnenlänge darstellt. Die Form der Ventilatorschaufeln, der profilierten (verzahnten) Kollisionsoberfläche und der Neigung sichern, daß die Ventilatorschaufeln mit dem Ausgangsmaterial abgefüllt werden und damit von der Abnutzungswirkung des Ausgangsmaterials geschützt werden, was auf die Verlängerung der Lebensdauer der Ventilatorschaufeln wirkt. Die Zähne 22b auf den Ventitatorschaufeln halten wie in Figur 6 die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Diese Ausführung hat im Vergleich zu obiger Ausführung (Fig. 6) den Vorteil, daß die Ventilatorschaufeln mit verschiedenen Neigungen (Winkel α) eingebaut werden können; somit ist der Austausch der Ventilatorschaufeln einfacher. Die Form der Ventilatorschaufeln hat keine Aussparung für die Stifte.

### Bezugszeichen

- 10: Vorrichtung
- 11: Gehäuse
- 11a: Gehäuseseite zur Materialeinfuhr
- 11b: Gehäuseseite mit Verschlußdeckel
- 11c: Verschlußdeckel
- 11d: Materialeinfuhrschacht
- 12: Rotorenscheiben
- 13: Motoren
- 13a: Riemen
- 14: Fundament / Gestell
- 15: Träger
- 16: Lager
- 17: Reckstangen
- 18: Einfuhrrohr
- 19: Auslaß
- 20: Kränze
- 20a: Ausnehmung
- 21: Stifte
- 22: Ventilatorschaufeln
- 22a: Kollisionsfläche
- 22b: Verzahnung
- 23: Kanäle
- 24: Zermahlenes Material
- 25: Drehrichtung
- 26: Luftströme

## Patentansprüche

1. Verfahren zur Herstellung von Ziegeln mit hoher Festigkeit, **gekennzeichnet durch** die folgenden Verfahrensschritte,
a) mikronisierter Wüstensand, welcher jeweils **durch** Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert wurde, wobei bis zu 28,36 % der Körner einen Durchmesser von weniger oder gleich 0,5 µm aufweisen, wird mit Kalk in einem Verhältnis von 0,906 - 0,954 : 0,046 - 0,094 (mikronisiertes Mineral:Kalk) zu einem Rohgemisch vermischt, wobei
b) anschließend dem Rohgemisch Wasser in einem Verhältnis 1 : 0,08 - 0,12 (Rohgemisch:Wasser) zugegeben wird, und
c) **durch** einen Druck zwischen 40 - 90 MPa und einer Temperatur zwischen 90 - 170 °C zwischen 4,5 und 7 Stunden ein Ziegel ausgehärtet wird.

2. Verfahren Anspruch 1, wobei die unbearbeiteten Rohstoffkomponenten einen bevorzugten Korngrößendurchmesser von bis zu 600 µm besitzen.

3. Verfahren nach einem der Ansprüche 1 oder 2, **dadurch gekennzeichnet, dass** der Kalk, der den mikronisierten Rohstoffkomponenten beigemengt wird, hydratisiert oder ungelöscht ist.

4. Verfahren nach einem der vorherigen Ansprüche, wobei das Formen des Ziegels in Verfahrensschritt 1 d) durch Pressen oder Vibrieren in Schablonen erfolgt.

## Claims

1. Method for the production of bricks with high stability, **characterized by** the following steps
a) micronized desert sand, which been reduced to small peaces by rotors equipped with plates on which ventilator blades are attached on one side and in which the ventilator blades are connected to the rings, reaching through in corresponding channels of the appropriate rotor plate across from it which prevent the loss of material under the ventilator blades, whereas up to 28,36 % of the small peaces have a diameter of less or equal than 0,5 µm, is admixed with lime in a ratio of 0,906 - 0,954 : 0,046 - 0,094 (mikronized mineral : lime) to a raw mixture, whereas
b) afterwards water in a ratio of 1 : 0,08 - 0,12 (raw mixture : water) is added,and
c) by a pressure of 40 - 90 MPa and a temperature between 90 - 170 °C for 4,5 to 7 hours a brick is hardened.

2. Method according to claim 1, where the untreated components of the raw mixture have a preferred diameter of the small pieces of up to 600 µm.

3. Method according to one of the claims 1 or 2, **characterized in, that** the lime, which is added to the components of the raw mixture, is hydrated or unslaked.

4. Method according to one of the previous claims, where the forming of the brick in step 1 d) is carried out by compression or vibration in the template.

## Revendications

1. Procédé pour fabriquer des briques de haute résistance, **caractérisé par** les étapes suivantes :
a) du sable boulant micronisé, qui a été broyé par des rotors constitués de disques sur lesquels les pales ou aubes de ventilateurs sont fixées d'un seul côté, lesdites pales ou aubes de ventilateur étant reliées aux couronnes et enfoncées dans des passages correspondants sur le disque de rotor qui leur fait chaque fois face, empêchant ainsi le passage de la matière sous les pales ou aubes de ventilateur, jusqu'à 28,36 % des grains présentant un diamètre inférieur ou égal à 0,5 µm, est mélangé à de la chaux dans une proportion de 0,906 - 0,954 : 0,046 - 0,094 (minéral micronisé : chaux) pour former un mélange brut,
b) de l'eau étant ensuite ajouté au mélange brut dans une proportion de 1 : 0,08 - 0,12 (mélange brut : eau), et
c) une brique étant durcie en la soumettant à une pression comprise entre 40 et 90 MPa et à une température de 90 à 170° C pendant 4,5 à 7 heures.

2. Procédé selon la revendication 1, sachant que les composants du mélange brut non traités ont un diamètre de grain préférentiel pouvant aller jusqu'à 600 µm.

3. Procédé selon l'une des revendication 1 ou 2, **caractérisé en ce que** la chaux qui est mélangée aux composants bruts micronisés est hydratée ou vive.

4. Procédé selon l'une des revendications précédentes, le façonnage de la brique à l'étape 1d) du procédé étant réalisé par pressage ou vibrage dans des moules.
